# EUROPEAN PATENT SPECIFICATION

(11) **EP 3 229 767 B1**
(45) Date of publication and mention of the grant of the patent: **29.01.2020**
(21) Application number: 15807941.8
(22) Date of filing: 10.12.2015
(51) Int. Cl.: A61K 8/37, A61K 8/44, A61K 8/46, A61Q 19/00, A61K 8/81, A61K 8/86, A61Q 19/10

(54) **CLEANSING COMPOSITION BASED ON A POLYETHYLENE GLYCOL AND A POLYETHER ESTER**
REINIGUNGSMITTEL ENTHALTEND EIN POLYETHYLENGLYCOL UND EIN POLYETHER-ESTER
COMPOSITION DE NETTOYAGE DE LA PEAU COMPRENANT UN POLYETHYLENE GLYCOL ET UN POLYETHER ESTER

(30) Priority: 10.12.2014 FR 1462197
(43) Date of publication of application: 18.10.2017
(73) Proprietor: L'Oréal, 75008 Paris (FR)
(72) Inventor: RAVAUD, Magali, 75011 Paris (FR); DELAHAIE, Alain, 94152 Chevilly Larue (FR)
(74) Representative: L'Oreal
(86) International application number: PCT/EP2015/079322
(87) International publication number: WO 2016/092048

(56) References cited:
- WO-A1-01/08643
- DE-A1-102007 059 703
- US-A- 5 192 462
- DATABASE WPI Week 200453 Thomson Scientific, London, GB; AN 2004-547639 XP002742380, & JP 2004 203776 A (YOUSKIN SEIYAKU KK) 22 July 2004 (2004-07-22)

## Description

The present invention relates to a composition comprising, especially in a physiologically acceptable medium:
(a) an aqueous phase; and
(b) at least one anionic foaming surfactant; and
(c) at least one amphoteric or zwitterionic foaming surfactant; and
(d) at least one PEG-90M and
(e) at least one of PEG-150 pentaerythrityl tetrastearate in isolated form or a mixture of PEG-150 pentaerythrityl tetrastearate, of PEG-6 caprylic/capric triglycerides and of water (50/20/30% by weight); and
(f) a specific polymeric suspension agent.

The present invention also relates to the uses of the same in cosmetics or dermatology, especially as products for cleansing human keratin materials and more particularly the skin.

Cleansing compositions for the body (shower gels, bath products), the hands (hand gels) or the face (facial foaming gels, makeup removers) are particularly appreciated by consumers. They are generally in the form of milks, creams or cream gels packaged in a tube, a bottle or a pump-action bottle.

These compositions have been described especially in the following documents:
- US 4 387 040; US 4 310 432 and US 310 433;
- Formulation Technology of Liquid Soaps by Eugene M. Franck - Cosmetics & Toiletries, Vol. 97, 1982, pages 49-54;
- Liquid Soap, a Challenge for the Formulator by Roger Hart - Household & Personal Care Products Industry, May 1981, pages 46-47;
- Comparison of Detergent Based versus Soap Based Liquid Soaps by Dennis W. Dyer and Thomas Hassapis - Soap/Cosmetics/Chemical Specialities for July, 1993, pages 36; 38 and 40;
- The Evolution of Liquid Soap by Larry Lundmark - Cosmetics & Toiletries, Vol. 107, December 1992, pages 49-52.

Cleansing formulations are particularly sought for the following advantages:
- they have good detergent properties and generally produce an airy foam which starts rapidly;
- they are hygienic by virtue of their packaging;
- they are easy to transport by virtue of their packaging;
- they are easy and economical to use.

These cleansing compositions for the body, the hands or the face, depending on the packaging, must have a suitable consistency and rheology to allow good uptake of the product by the user and good spreading onto the skin surface to be washed.

Many cleansing product formulations currently on the market have a tendency to produce a very airy foam by virtue of the size of the air bubbles. As a result, the user has a very low physical and visual perception of this type of foam, which tends to disappear rapidly on contact of the product with the skin. These formulations also have a tendency to give an unpleasant dragging sensation on spreading (lack of glidance) and the softness experienced by the user after rinsing and drying is not entirely satisfactory.

The need thus remains to find novel compositions for cleansing keratin materials of the body, the face and the hands, based on foaming surfactants and a suitable thickening system, which:
1/ have better glidance on spreading without the drawbacks listed previously;
2/ produce a denser, more compact, creamier and immediately physically and visually perceptible foam;
3/ leave on the keratin material an excellent sensation of softness after rinsing and drying, especially leaving the skin soft, silky and velvety (velvet effect) and/or a powdery finish;
4/ are easy to remove on rinsing and leave a soft and protective film on the skin.

The Applicants have discovered, surprisingly, that these objectives can be met by using a composition according to claim 1.

This discovery forms the basis of the invention.

The present invention relates to a cosmetic process for treating a keratin material, which consists in applying to the surface of said keratin material a composition as defined previously, followed by rinsing with water.

The present invention more particularly relates to a cosmetic process for cleansing a keratin material, which consists in applying to the surface of the keratin material a composition as defined previously, followed by rinsing with water.

For the purposes of the present invention, the term "physiologically acceptable medium" is intended to denote a medium that is suitable for the topical administration of a composition. A physiologically acceptable medium is a medium which has no unpleasant odor and/or appearance, and which is perfectly compatible with topical administration.

The term "keratin material" means the skin, the scalp, the lips and/or integuments such as the nails and keratin fibres, for instance bodily hair, the eyelashes, the eyebrows and head hair.

For the purposes of the present invention, the term "polymeric compound" means any molecule, macromolecule or compound comprising a repetition of units known as monomers (at least three monomers) that are linked together via covalent bonding by a chemical reaction.

The compositions are preferably "rinse-off' compositions (rinsing with water or a tonic lotion after application) and may be used, for example, for removing makeup and cleansing the face, or cleansing the body, the hair, the scalp and mucous membranes such as the lips. They may constitute care products, for instance rinse-off masks.

### POLYALKYLENE GLYCOL

The polyalkylene glycol in accordance with the present invention is PEG 90M (n = 90000) such as the product sold under the trade name Polyox WSR-301® (The Dow Chemical Company).

The polyethylene glycol(s) are preferably present in the composition in concentrations ranging from 0.01% to 1% and more preferentially from 0.05% to 0.5% by weight relative to the total weight of the composition.

### POLYETHER ESTER

The polyether ester in accordance with the invention. is PEG-150 pentaerythrityl tetrastearate in isolated form, such as the commercial product Crothix PA® (MH) from Croda or a mixture of PEG-150 pentaerythrityl tetrastearate, of PEG-6 caprylic/capric triglycerides and of water (50/20/30% by weight) such as the commercial product Crothix LQ® (MH) from Croda.

The polyether ester(s) are preferably present in the composition as active material in concentrations ranging from 0.01% to 2% and more preferentially from 0.05% to 1% by weight relative to the total weight of the composition.

Preferably, the polyether ou polyether ester(s) are present in the composition in concentrations ranging from 0.01% to 1% in weight and more preferentially from 0.05% to 0,5% by weight relative to the total weight of the composition.

### FOAMING SURFACTANT

Foaming surfactants are detergents and differ from emulsifiers in the value of their HLB (Hydrophilic-Lipophilic Balance), the HLB being the ratio of the hydrophilic part to the lipophilic part in the molecule. The term "HLB" is well known to those skilled in the art and is described, for example, in "The HLB system. A time-saving guide to Emulsifier Selection" (published by ICI Americas Inc., 1984). For emulsifiers, the HLB generally ranges from 3 to 8 for the preparation of W/O emulsions and from 8 to 18 for the preparation of O/W emulsions, whereas foaming surfactants generally have an HLB of greater than 20. The HLB or hydrophilic-lipophilic balance of the surfactant(s) used according to the invention may be determined via the Griffin method or the Davies method.

### ANIONIC FOAMING SURFACTANTS

The anionic surfactants that may be present in the composition according to the invention may be chosen especially from anionic derivatives of proteins of plant origin or of silk proteins, phosphates and alkyl phosphates, carboxylates, sulfosuccinates, amino acid derivatives, alkyl sulfates, alkyl ether sulfates, sulfonates, isethionates, taurates, alkyl sulfoacetates, polypeptides, anionic derivatives of alkyl polyglucoside, soaps (fatty acid salts), and mixtures thereof.
a) Anionic derivatives of proteins of plant origin are protein hydrolysates bearing a hydrophobic group, it being possible for said hydrophobic group to be naturally present in the protein or to be added by reaction of the protein and/or of the protein hydrolysate with a hydrophobic compound. The proteins are of plant origin or are derived from silk, and the hydrophobic group may in particular be a fatty chain, for example an alkyl chain comprising from 10 to 22 carbon atoms. As anionic derivatives of proteins of plant origin, mention may more particularly be made of apple, wheat, soybean or oat protein hydrolysates comprising an alkyl chain containing from 10 to 22 carbon atoms, and salts thereof. The alkyl chain may especially be a lauryl chain and the salt may be a sodium, potassium and/or ammonium salt.
   Thus, as protein hydrolysates bearing a hydrophobic group, mention may be made, for example, of salts of protein hydrolysates where the protein is a silk protein modified with lauric acid, such as the product sold under the name Kawa Silk by Kawaken; salts of protein hydrolysates where the protein is a wheat protein modified with lauric acid, such as the potassium salt sold under the name Aminofoam W OR by Croda (CTFA name: potassium lauroyl wheat amino acids) and the sodium salt sold under the name Proteol LW 30 by the company SEPPIC (CTFA name: sodium lauroyl wheat amino acids); salts of protein hydrolysates where the protein is an oat protein comprising an alkyl chain containing from 10 to 22 carbon atoms and more especially salts of protein hydrolysates where the protein is an oat protein modified with lauric acid, such as the sodium salt sold under the name Proteol OAT (30% aqueous solution) by the company SEPPIC (CTFA name: sodium lauroyl oat amino acids); or salts of apple protein hydrolysates comprising an alkyl chain containing from 10 to 22 carbon atoms, such as the sodium salt sold under the name Proteol APL (30% aqueous glycol solution) by the company SEPPIC (CTFA name: sodium cocoyl apple amino acids). Mention may also be made of the mixture of lauroyl amino acids (aspartic acid, glutamic acid, glycine, alanine) neutralized with sodium N-methylglycinate sold under the name Proteol SAV 50 S by the company SEPPIC (CTFA name: sodium cocoyl amino acids).
b) Examples of phosphates and alkyl phosphates that may be mentioned include monoalkyl phosphates and dialkyl phosphates, such as the lauryl monophosphate sold under the name MAP 20® by the company Kao Chemicals, the potassium salt of dodecylphosphoric acid, mixture of monoester and diester (predominantly diester) sold under the name Crafol AP-31® by the company Cognis, the mixture of octylphosphoric acid monoester and diester sold under the name Crafol AP-20® by the company Cognis, the mixture of ethoxylated (7 mol of EO) phosphoric acid monoester and diester of 2-butyloctanol, sold under the name Isofol 12 7 EO-Phosphate Ester® by the company Condea, the potassium or triethanolamine salt of mono(C12-C13)alkyl phosphate sold under the references Arlatone MAP 230K-40® and Arlatone MAP 230T-60® by the company Uniqema, the potassium lauryl phosphate sold under the name Dermalcare MAP XC-99/09® by the company Rhodia Chimie, and the potassium cetyl phosphate sold under the name Arlatone MAP 160K by the company Uniqema.
c) As carboxylates, mention may be made of:
   amido ether carboxylates (AEC), such as sodium lauryl amido ether carboxylate (3 EO), sold under the name Akypo Foam 30® by the company Kao Chemicals;
   polyoxyethylenated carboxylic acid salts, such as oxyethylenated (6 EO) sodium lauryl ether carboxylate (65/25/10 C12-C14-C16), sold under the name Akypo Soft 45 NV® by the company Kao Chemicals, polyoxyethylenated and carboxymethylated fatty acids originating from olive oil, sold under the name Olivem 400® by the company Biologia E Tecnologia, or oxyethylenated (6 EO) sodium tridecyl ether carboxylate, sold under the name Nikkol ECTD-6NEX® by the company Nikkol;
   salts of fatty acids (soaps) containing a C6 to C22 alkyl chain which are neutralized with an organic or mineral base, such as potassium hydroxide, sodium hydroxide, triethanolamine, N-methylglucamine, lysine and arginine.
d) Amino acid derivatives that may especially be mentioned include alkaline salts of amino acids, such as:
   sarcosinates, for instance sodium lauroyl sarcosinate, sold under the name Sarkosyl NL 97® by the company Ciba or sold under the name Oramix L 30® by the company SEPPIC, sodium myristoyl sarcosinate, sold under the name Nikkol Sarcosinate MN® by the company Nikkol, or sodium palmitoyl sarcosinate, sold under the name Nikkol Sarcosinate PN® by the company Nikkol; alaninates, such as sodium N-lauroyl-N-methylamidopropionate, sold under the name Sodium Nikkol Alaninate LN 30® by the company Nikkol or sold under the name Alanone Ale® by the company Kawaken, and triethanolamine N-lauroyl-N-methylalanine, sold under the name Alanone Alta® by the company Kawaken;
   glutamates, for instance triethanolamine monococoyl glutamate sold under the name Acylglutamate CT-12® by the company Ajinomoto, triethanolamine lauroyl glutamate sold under the name Acylglutamate LT-12® by the company Ajinomoto, aspartates, for instance the mixture of triethanolamine N-lauroyl aspartate/triethanolamine N-myristoyl aspartate sold under the name Asparack® by the company Mitsubishi, glycine derivatives (glycinates), for instance sodium N-cocoyl glycinate sold under the names Amilite GCS-12® and Amilite GCK 12 by the company Ajinomoto;
   citrates, such as the oxyethylenated (9 mol) citric monoester of cocoyl alcohols sold under the name Witconol EC 1129 by the company Goldschmidt, and galacturonates such as sodium dodecyl D-galactoside uronate sold by the company Soliance.
e) Examples of sulfosuccinates that may be mentioned include the oxyethylenated (3 EO) lauryl alcohol monosulfosuccinate (70/30 C12/C14) sold under the names Setacin 103 Special® and Rewopol SB-FA 30 K 4® by the company Witco, the disodium salt of a hemisulfosuccinate of C12-C14 alcohols, sold under the name Setacin F Special Paste® by the company Zschimmer Schwarz, the oxyethylenated (2 EO) disodium oleamidosulfosuccinate sold under the name Standapol SH 135® by the company Cognis, the oxyethylenated (5 EO) laurylamide monosulfosuccinate sold under the name Lebon A-5000® by the company Sanyo, the oxyethylenated (10 EO) disodium salt of lauryl citrate monosulfosuccinate sold under the name Rewopol SB CS 50® by the company Witco, and the ricinoleic monoethanolamide monosulfosuccinate sold under the name Rewoderm S 1333® by the company Witco. Polydimethylsiloxane sulfosuccinates may also be used, such as disodium PEG-12 dimethicone sulfosuccinate sold under the name Mackanate-DC30 by the company MacIntyre.
f) Examples of alkyl sulfates that may be mentioned include triethanolamine lauryl sulfate (CTFA name: TEA lauryl sulfate), such as the product sold by Huntsman under the name Empicol TL40 FL or the product sold by Cognis under the name Texapon T42, which products are at 40% in aqueous solution. Mention may also be made of ammonium lauryl sulfate (CTFA name: ammonium lauryl sulfate), such as the product sold by Huntsman under the name Empicol AL 30FL, which is at 30% in aqueous solution.
g) Examples of alkyl ether sulfates that may be mentioned include sodium lauryl ether sulfate (CTFA name: sodium laureth sulfate), such as the product sold under the names Texapon N40 and Texapon AOS 225 UP by the company Cognis, or ammonium lauryl ether sulfate (CTFA name: ammonium laureth sulfate), such as the product sold under the name Standapol EA-2 by the company Cognis.
h) Examples of sulfonates that may be mentioned include α-olefinsulfonates, such as the sodium α-olefinsulfonate (C14-C16), sold under the name Bio-Terge AS-40® by the company Stepan, sold under the names Witconate AOS Protégé® and Sulframine AOS PH 12® by the company Witco or sold under the name Bio-Terge AS-40 CG® by the company Stepan, secondary sodium olefinsulfonate, sold under the name Hostapur SAS 30® by the company Clariant; or linear alkylarylsulfonates, such as sodium xylenesulfonate, sold under the names Manrosol SXS30®, Manrosol SXS40® and Manrosol SXS93® by the company Manro.
i) Isethionates that may be mentioned include acylisethionates, for instance sodium cocoyl isethionate, such as the product sold under the name Jordapon CI P® by the company Jordan.
j) Taurates that may be mentioned include the sodium salt of palm kernel oil methyltaurate sold under the name Hostapon CT Pate® by the company Clariant; N-acyl N-methyltaurates, for instance the sodium N-cocoyl N-methyltaurate sold under the name Hostapon LT-SF® by the company Clariant or sold under the name Nikkol CMT-30-T® by the company Nikkol, and the sodium palmitoyl methyltaurate sold under the name Nikkol PMT® by the company Nikkol.
k) The anionic derivatives of alkyl polyglucosides may especially be citrates, tartrates, sulfosuccinates, carbonates and ethers of glycerol obtained from alkyl polyglucosides. Mention may be made, for example, of the sodium salt of cocoyl polyglucoside (1,4) tartaric ester, sold under the name Eucarol AGE-ET® by the company Cesalpinia, the disodium salt of cocoyl polyglucoside (1,4) sulfosuccinic ester, sold under the name Essai 512 MP® by the company SEPPIC, or the sodium salt of cocoyl polyglucoside (1,4) citric ester, sold under the name Eucarol AGE-EC® by the company Cesalpinia.
l) The soaps are obtained from a fatty acid which is partially or completely saponified (neutralized) with a basic agent. They are alkali metal or alkaline-earth metal soaps or soaps of organic bases. Use may be made, as fatty acids, of saturated, linear or branched fatty acids comprising from 8 to 30 carbon atoms and preferably comprising from 8 to 22 carbon atoms. This fatty acid may be chosen in particular from palmitic acid, stearic acid, myristic acid and lauric acid, and mixtures thereof.

Examples of basic agents that may be used include alkali metal hydroxides (sodium hydroxide and potassium hydroxide), alkaline-earth metal hydroxides (for example magnesium hydroxide), ammonium hydroxide or else organic bases, such as triethanolamine, N-methylglucamine, lysine and arginine.

The soaps may especially be fatty acid alkali metal salts, the basic agent being an alkali metal hydroxide and preferably potassium hydroxide (KOH).

The amount of basic agent must be sufficient for the fatty acid to be at least partially neutralized.

Preferably, the anionic surfactant is chosen from alkyl sulfates, alkyl ether sulfates such as sodium lauryl ether sulfate, isethionates, amino acid derivatives, in particular glycine derivatives (glycinates), for instance sodium N-cocoyl glycinate and mixtures thereof, and even more particularly alkyl ether sulfates such as sodium lauryl ether sulfate.

The anionic surfactant(s) are preferably present in concentrations ranging from 5% to 15% by weight of active material and more preferentially from 8% to 12% by weight relative to the total weight of the formulation.

### AMPHOTERIC AND ZWITTERIONIC FOAMING SURFACTANTS

The amphoteric and zwitterionic surfactants may be chosen, for example, from betaines, N-alkylamido betaines and derivatives thereof, sultaines, alkyl polyaminocarboxylates, alkylamphoacetates, and mixtures thereof.

Betaines that may especially be mentioned include alkyl betaines, for instance cocoyl betaine, such as the product sold under the name Dehyton AB-30® by the company Cognis, lauryl betaine, such as the product sold under the name Genagen KB® by the company Clariant, oxyethylenated (10 EO) lauryl betaine, such as the product sold under the name Lauryl Ether (10 EO) Betaine® by the company Shin Nihon Rica, or oxyethylenated (10 EO) stearyl betaine, such as the product sold under the name Stearyl Ether (10 EO) Betaine® by the company Shin Nihon Rica.

Among the N-alkylamido betaines and derivatives thereof, examples that may be mentioned include the cocamidopropyl betaine sold under the name Lebon 2000 HG® by the company Sanyo or sold under the name Empigen BB® by the company Albright & Wilson, or the lauramidopropyl betaine sold under the name Rewoteric AMB12P® by the company Witco.

Sultaines that may be mentioned include hydroxysultaines, such as cocamidopropyl hydroxysultaine, for instance the product sold under the name Rewoteric AM CAS by the company Goldschmidt-Degussa or the product sold under the name Crosultaine C-50® by the company Croda.

Alkyl polyaminocarboxylates (APACs) that may be mentioned include sodium cocoylpolyaminocarboxylate, sold under the names Ampholak 7 CX/C® and Ampholak 7 CX® by the company Akzo Nobel, sodium stearylpolyamidocarboxylate, sold under the name Ampholak 7 TX/C by the company Akzo Nobel, or sodium carboxymethyloleylpolypropylamine, sold under the name Ampholak XO7/C® by the company Akzo Nobel.

Examples of alkylamphoacetates that may be mentioned include N-disodium N-cocoyl-N-carboxymethoxyethyl-N-(carboxymethyl)ethylenediamine (CTFA name: disodium cocoamphodiacetate), such as the product sold under the name Miranol C2M Concentrate NP® by the company Rhodia, N-sodium N-cocoyl-N-hydroxyethyl-N-(carboxymethyl)ethylenediamine (CTFA name: sodium cocamphoacetate) or sodium cocoamphohydroxypropylsulfonate, sold under the name Miranol CSE® by the company Rhodia.

Preferably, the amphoteric or zwitterionic foaming surfactant is chosen from betaines, and especially alkyl betaines and N-alkylamido betaines, and mixtures thereof, alkyl amphoacetates such as sodium cocoamphoacetate, and mixtures thereof, and even more particularly from cocoyl betaine and cocamidopropyl betaine, and mixtures thereof.

The amphoteric or zwitterionic surfactant(s) are preferably present in concentrations ranging from 1% to 10% by weight of active material and more preferentially from 1% to 5% by weight relative to the total weight of the formulation.

According to a particular form of the invention, the compositions may also contain one or more nonionic surfactants.

### Nonionic foaming surfactant

The nonionic foaming surfactants that may be present in the composition of the invention may be chosen especially from alkyl polyglucosides (APG), oxyalkylenated glycerol esters and oxyalkylenated sugar esters, and mixtures thereof. They are preferably APGs.

Use is preferably made, as alkyl polyglucosides, of those containing an alkyl group comprising from 6 to 30 carbon atoms and preferably from 8 to 16 carbon atoms and containing a glucoside group preferably comprising from 1.2 to 3 glucoside units. The alkylpolyglucosides may be chosen, for example, from decylglucoside (alkyl-C9/C11-polyglucoside (1.4)), for instance the product sold under the name Mydol 10® by the company Kao Chemicals or the product sold under the name Plantacare 2000 UP® by the company Cognis; caprylyl/capryl glucoside, for instance the product sold under the name Plantacare KE 3711® by the company Cognis; laurylglucoside, for instance the product sold under the name Plantacare 1200 UP® by the company Cognis; cocoyl glucoside, for instance the product sold under the name Plantacare 818 UP® by the company Cognis; caprylylglucoside, for instance the product sold under the name Plantacare 810 UP® by the company Cognis; and mixtures thereof.

The oxyalkylenated glycerol esters are especially polyoxyethylenated derivatives of esters of glycerol and of a fatty acid and of their hydrogenated derivatives. These oxyalkylenated glycerol esters may be chosen, for example, from glyceryl esters of fatty acids which are hydrogenated and oxyethylenated, such as PEG-200 hydrogenated glyceryl palmate, sold under the name Rewoderm LI-S 80® by the company Goldschmidt; oxyethylenated glyceryl cocoates, such as PEG-7 glyceryl cocoate, sold under the name Tegosoft GC® by the company Goldschmidt, and PEG-30 glyceryl cocoate, sold under the name Rewoderm LI-63 by the company Goldschmidt; and mixtures thereof.

The oxyalkylenated sugar esters are especially polyethylene glycol ethers of fatty acid and sugar esters. These oxyalkylenated sugar esters may be chosen, for example, from oxyethylenated glucose esters, such as PEG-120 methyl glucose dioleate, sold under the name Glucamate DOE 120® by the company Amerchol.

According to a preferred embodiment of the invention, the nonionic surfactant is an alkyl polyglucoside which may be chosen especially from decyl glucoside, caprylyl/capryl glucoside, lauryl glucoside, cocoyl glucoside and caprylyl glucoside, and mixtures thereof.

The nonionic surfactant(s) are preferably present in concentrations ranging from 0.5% to 10% by weight of active material, more preferentially from 0.5% to 6% by weight and more particularly from 0.5% to 2% by weight relative to the total weight of the formulation.

### POLYMERIC SUSPENSION AGENT

The polymeric suspension agents may be of natural origin. They may be chosen from water-soluble polysaccharides.

The term "polysaccharide" means any polymer formed from several saccharides (or monosaccharides) of general formula: -[Cₓ(H₂O)_{y})]ₙ- (in which y is generally x-1), which are linked together via O-oside linkages.

The term "water-soluble polysaccharide" means any polysaccharide which, when introduced into water or a mixture of water and of linear or branched C₂-C₅ monoalcohol, for example ethanol, isopropanol or N-propanol, without modification of the pH at 25°C, at a weight concentration equal to 1%, makes it possible to form a macroscopically homogeneous and transparent solution: homogeneous and transparent solution, i.e. a solution with a light transmission value, at a wavelength of 500 nm, through a sample 1 cm thick, of at least 80% and preferably at least 90%.

The water-soluble polysaccharides that may be used according to the invention are chosen in particular from starches, carrageenans, xanthan gums, guar gums, celluloses such as hydroxyethylcellulose and hydroxypropylcellulose, and mixtures thereof.

The polymeric suspension agents may be of synthetic origin.

They may be chosen from:
- associative or non-associative crosslinked homopolymers and crosslinked copolymers of acrylic acid and/or methacrylic acid and/or esters thereof;
- associative polyether-urethane polymers.

For the purposes of the present invention, the term "associative polymer" means any hydrophilic polymer that is capable, in an aqueous medium, of reversibly combining with itself or with other molecules. Its chemical structure in particular comprises at least one hydrophilic portion and at least one hydrophobic portion.

The term "hydrophobic group" means a radical or polymer comprising a saturated or unsaturated, linear or branched hydrocarbon-based chain. When the hydrophobic group is a hydrocarbon-based radical, it generally comprises at least 10 carbon atoms, preferably from 10 to 30 carbon atoms, more particularly from 12 to 30 carbon atoms and even more particularly from 18 to 30 carbon atoms. Preferably, the hydrocarbon-based hydrophobic group is derived from a monofunctional compound.

For example, the functional group may be derived from a fatty alcohol, such as stearyl alcohol, dodecyl alcohol or decyl alcohol, or from a polyoxyalkylenated fatty alcohol, such as Steareth-100. It may also denote a hydrocarbon-based polymer, for instance a polybutadiene.

For the purposes of the present invention, the term "non-associative polymer" means any polymer which does not have the behaviour of an associative polymer as explained previously. It is generally a hydrophilic polymer of acrylic acid and/or methacrylic acid and of an ester thereof comprising less than 6 carbon atoms: i.e. a C₁-C₄ alkyl acrylate chosen, for example, from methyl acrylate, ethyl acrylate and butyl acrylate.

The copolymers according to the invention may be partially or totally crosslinked with at least one crosslinking agent which may be chosen from polyethylenically unsaturated compounds, and in particular polyalkenyl ethers of sucrose, polyalkenyl ethers of polyols, diallyl phthalates, divinylbenzene, allyl (meth)acrylate, ethylene glycol di(meth)acrylate, methylenebisacrylamide, trimethylolpropane tri(meth)acrylate, diallyl itaconate, diallyl fumarate, diallyl maleate, zinc (meth)acrylate, castor oil derivatives and polyol derivatives manufactured from unsaturated carboxylic acids.

Examples of such polymers that may be mentioned include:
(1) acrylic acid homopolymers, crosslinked with pentaerythrityl allyl ether, a sucrose allyl ether or a propylene glycol allyl ether, for instance the Carbomers sold under the trade name Carbopol® by the company Lubrizol;
(2) non-associative crosslinked copolymers of acrylic acid and/or methacrylic acid and/or esters thereof.

Examples of such polymers that may be mentioned include:
i) non-associative crosslinked copolymers of acrylic acid and/or methacrylic acid and an ester thereof comprising less than 6 carbon atoms, and more particularly in the form of an aqueous 28% dispersion, such as the copolymer sold under the trade name Aculyn 33® by The Dow Chemical Company and having the INCI name: Acrylates copolymer;
ii) crosslinked copolymers comprising at least one methacrylic acid unit and at least one C₁-C₄ alkyl acrylate unit. These copolymers are especially described in patent application WO 01/76552.

In these crosslinked copolymers described in said document, the methacrylic acid units may be present, for example, in an amount ranging from 20% to 80% by weight, such as from 25% to 70% by weight and more particularly from 35% to 60% by weight, relative to the total weight of the copolymer.

In these crosslinked copolymers described in said document, the C₁-C₄ alkyl acrylate units may be present, for example, in an amount ranging from 15% to 80% by weight, such as from 25% to 75% by weight and more particularly from 40% to 65% by weight, relative to the total weight of the copolymer. The alkyl acrylate unit may be chosen, for example, from methyl acrylate, ethyl acrylate and butyl acrylate. According to a particular mode, the alkyl acrylate is ethyl acrylate.

According to a particular form of the invention, the crosslinked copolymer may be in the form of a dispersion in water. The number-average size of the copolymer particles in the dispersion is generally, for example, from 10 to 500 nm, or even from 20 to 200 nm and in particular from 50 to 150 nm.

Use may be made in particular of a crosslinked copolymer of methacrylic acid and of ethyl acrylate in the form of an aqueous 30% dispersion manufactured and sold under the trade name Carbopol Aqua SF-1® by the company Noveon.

Among the polymeric suspension agents chosen from crosslinked homopolymers or copolymers of acrylic acid and/or of methacrylic acid and/or of an ester thereof, the following may be mentioned:
(3) crosslinked associative polymers of acrylic acid and/or of methacrylic acid and/or of an ester thereof, and more particularly those formed from 95% to 60% by weight of acrylic acid (hydrophilic unit), 4% to 40% by weight of C₁₀-C₃₀ alkyl acrylate (hydrophobic unit) and 0 to 6% by weight of crosslinking monomer, or alternatively those formed from 98% to 96% by weight of acrylic acid (hydrophilic unit), 1% to 4% by weight of C₁₀-C₃₀ alkyl acrylate (hydrophobic unit) and 0.1% to 0.6% by weight of crosslinking polymerizable monomer such as those mentioned previously.

Among these polymers, mention may be made of the products sold by the company Goodrich under the brand names Pemulen TR1®, Pemulen TR2® and Carbopol 1382®, and more preferentially Pemulen TR1®, and the product sold by the company SEPPIC under the name Coatex SX® and having the INCI name: Acrylates/C10-C30 Alkyl Acrylate Crosspolymer.

Mention may also be made of copolymers comprising among their monomers an α,β-monoethylenically unsaturated carboxylic acid and an ester of an α,β-monoethylenically unsaturated carboxylic acid and of an oxyalkylenated fatty alcohol. Preferably, these compounds also comprise, as monomer, an ester of a C₁-C₄ alcohol and of an α,β-monoethylenically unsaturated carboxylic acid. For example, mention may be made of the product Aculyn 22® sold by The Dow Chemical Company, which is a terpolymer of polyoxyethylene stearyl methacrylate/ethyl acrylate/methacrylic acid having the INCI name Acrylates/Steareth-20 Methacrylate copolymer.

Mention may also be made of crosslinked copolymers of acrylic acid/vinyl isodecanoate, such as the product sold under the trade name Stabylen 30® (INCI name: Acrylates/vinyl isodecanoate crosspolymer) sold by the company Sigma 3V.

Mention may also be made of copolymers of vinyl neodecanoate and of one or more acrylic acid or methacrylic acid monomers or an ester thereof; said copolymers being crosslinked with a trimethylolpropane or pentaerythrityl alkyl ether, such as the product sold under the trade name Aculyn 38® (INCI name: Acrylates/vinyl neodecanoate crosspolymer) sold by The Dow Chemical Company.

Another family of polymeric suspension agents that may be used in the compositions of the invention comprises associative nonionic polyether-urethanes.

Associative nonionic polyether-urethanes are block copolymers comprising in the chain both hydrophilic blocks generally of the polyoxyethylene type (polyurethanes may also be termed polyurethane polyethers) and hydrophobic blocks, which may be solely aliphatic blocks and/or cycloaliphatic blocks and/or aromatic blocks.

In particular, these polymers comprise at least two hydrocarbon-based lipophilic chains containing from 6 to 30 carbon atoms, said chains possibly being separated by a hydrophilic block or placed at the end of the hydrophilic block. In particular, it is possible for one or more pendent chains to be envisaged. In addition, the polymer may comprise a hydrocarbon-based chain at one or both ends of a hydrophilic block.

The associative polyurethanes may be in the form of block polymers, as triblocks or multi-blocks. The hydrophobic blocks may thus be at each end of the chain (for example, the triblock copolymer contains a hydrophilic central block) or distributed both at the ends and in the chain (for example: multiblock polymers). These polymers may also be grafted polymers or starburst polymers. Preferably, the associative polyurethanes are triblock polymers in which the hydrophilic block is a polyoxyethylene chain comprising from 50 to 1000 ethylene oxide groups. In general, the associative polyurethanes comprise a urethane bond between blocks.

As examples of fatty-chain nonionic polyurethane polyethers according to the invention, mention may be made of the products Rheolate® FX 1100 (Steareth-100/PEG 136/HDI (hexamethyl diisocyanate) crosslinked copolymer) and Rheolate® 205® containing a urea function, sold by the company Elementis, or the products Rheolate® 208, 204 or 212.

Mention may also be made of the product Elfacos T210® containing a C₁₂-C₁₄ alkyl chain, and the product Elfacos T212® containing a C₁₆-C₁₈ alkyl chain (PPG-14 Palmeth-60 Hexyl Dicarbamate), from Akzo.

The associative polyurethanes that may be used according to the invention are in particular described in the article by G. Fonnum, J. Bakke and Fk. Hansen - Colloid Polym. Sci., 271, 380-389 (1993).

According to a particular form of the invention, use may be made of a polyurethane polyether that may be obtained by polycondensation of at least three compounds comprising (i) at least one polyethylene glycol comprising from 150 to 180 mol of ethylene oxide, (ii) stearyl alcohol or decyl alcohol, and (iii) at least one diisocyanate.

Such polyether-urethanes are sold in particular by The Dow Chemical Company under the names Aculyn 46® and Aculyn 44®. Aculyn 46® is a polycondensate of polyethylene glycol containing 150 or 180 mol of ethylene oxide, of stearyl alcohol and of methylenebis(4-cyclohexyl isocyanate) (SMDI), at 15% by weight in a mixture of maltodextrin (4%) and water (81%), and Aculyn 44® is a polycondensate of polyethylene glycol containing 150 or 180 mol of ethylene oxide, of decyl alcohol and of methylenebis(4-cyclohexyl isocyanate) (SMDI), at 35% by weight in a mixture of propylene glycol (39%) and water (26%).

Use will be made more particularly of associative nonionic polyurethane polyethers such as the product sold by the company Elementis under the name Rheolate FX 1100®, which is a polycondensate of polyethylene glycol containing 136 mol of ethylene oxide, of stearyl alcohol polyoxyethylenated with 100 mol of ethylene oxide and hexamethylene diisocyanate (HDI) with a weight-average molecular weight of 30 000 (INCI name: PEG-136/Steareth-100l/HDI Copolymer).

According to a particularly preferred embodiment of the invention, the polymeric suspension agents will be chosen from crosslinked non-associative copolymers of acrylic acid and/or methacrylic acid and esters thereof and more particularly:
- crosslinked copolymers comprising at least one methacrylic acid unit and at least one ethyl acrylate unit and more particularly in the form of an aqueous 30% dispersion, such as the commercial product manufactured and sold under the name Carbopol Aqua SF-1® by the company Noveon;
- crosslinked non-associative copolymers of acrylic acid and/or methacrylic acid and an ester thereof comprising less than 6 carbon atoms, and more particularly in the form of an aqueous 28% dispersion, such as the product sold under the trade name Aculyn 33® by The Dow Chemical Company and having the INCI name: Acrylates Copolymer, and mixtures thereof.

The suspension agent may preferably be included in the composition of the invention in a concentration ranging from 0.1% to 10% by weight of active material relative to the total weight of the composition, more preferentially from 1% to 8% by weight and more particularly from 2% to 5% by weight relative to the total weight of the composition.

### ADDITIVES

The composition according to the invention may contain various additives chosen from those conventionally used in products for cleansing keratin materials, provided that these additives and the amounts thereof do not harm the desired qualities for the composition according to the invention.

For the purposes of the present invention, a "cosmetic additive" is a compound intended to impart a cosmetic property, which may be chosen, for example, from: provision of colour, texture modification, skin moisturization, visual and/or sensory properties, etc.

The compositions of the invention may comprise adjuvants usually used in the cosmetics field and especially those used in cleansing products. Examples of adjuvants that may be mentioned include fragrances, preserving agents, sequestrants (EDTA), pigments, nacreous agents or opacifiers, mineral or organic, matt-effect, bleaching or exfoliant fillers, soluble dyes, cosmetic or dermatological active agents, and fatty substances that are incompatible with the aqueous medium, such as oils or waxes.

The composition may also comprise a polymeric quaternary ammonium salt (different from the preceding surfactants).

These compounds are conditioning agents, i.e. they make it possible to increase the amount of foam and to produce a sensation of softness and comfort on the skin (moisturization maintenance).

Polymeric quaternary ammonium salts are cationic or amphoteric polymers containing at least one quaternized nitrogen atom. Polymeric quaternary ammonium salts that may especially be mentioned include the Polyquaternium products (CTFA name), which afford softness and creaminess to the foaming cream. These polymers may preferably be chosen from the following polymers:
Polyquaternium 5, such as the product Merquat 5 sold by the company Nalco;
Polyquaternium 6, such as the product Salcare SC 30 sold by the company Ciba, and the product Merquat 100 sold by the company Nalco;
Polyquaternium 7, such as the products Merquat S, Merquat 2200 and Merquat 550 sold by the company Nalco, and the product Salcare SC 10 sold by the company Ciba;
Polyquaternium 10, such as the product Polymer JR400 sold by the company Amerchol;
Polyquaternium 11, such as the products Gafquat 755, Gafquat 755N and Gafquat 734 sold by the company ISP;
Polyquaternium 15, such as the product Rohagit KF 720 F sold by the company Röhm;
Polyquaternium 16, such as the products Luviquat FC905, Luviquat FC370, Luviquat HM552 and Luviquat FC550 sold by the company BASF;
Polyquaternium 22, such as the product Merquat 280 sold by the company Nalco;
Polyquaternium 28, such as the product Styleze CC10 sold by the company ISP;
Polyquaternium 39, such as the products Merquat Plus 3330 and Merquat 3330PR sold by the company Nalco;
Polyquaternium 44, such as the product Luviquat Care sold by the company BASF;
Polyquaternium 46, such as the product Luviquat Hold sold by the company BASF;
Polyquaternium 47, such as the product Merquat 2001 sold by the company Nalco.

Preferably, the quaternary ammonium salts are chosen from Polyquaternium-7, Polyquaternium-10, Polyquaternium-39 and Polyquaternium-47, and mixtures thereof.

The polymeric quaternary ammonium salts may be in an (active material) amount ranging, for example, from 0.01% to 5% by weight and better still from 0.05% to 1% by weight relative to the total weight of the composition.

An example of a particular conditioning agent that may be mentioned is Polyquaternium-39, sold especially by the company Nalco under the names Merquat Plus 3330 and Merquat 3330PR.

The composition according to the invention comprises an aqueous medium or aqueous phase, i.e. a medium comprising an amount of water of at least 50% by weight, preferably ranging from 50% to 95% by weight and better still from 60% to 90% by weight, relative to the total weight of the composition.

The aqueous phase of the compositions according to the invention may contain, besides water, one or more solvents chosen from monoalcohols comprising from 1 to 6 carbon atoms, and polyols, and mixtures thereof. A monoalcohol that may especially be mentioned is ethanol. Polyols that may especially be mentioned include glycerol; glycols such as butylene glycol, isoprene glycol or propylene glycol; sorbitol; sugars such as glucose, fructose, maltose, lactose and sucrose; and mixtures thereof.

Examples of oils that may be mentioned include oils of plant origin (jojoba, avocado, sesame, sunflower, corn, soybean, safflower or grapeseed), mineral oils (petroleum jelly, optionally hydrogenated isoparaffins), synthetic oils (isopropyl myristate, cetearyl octanoate, polyisobutylene, ethylhexyl palmitate, alkyl benzoates), volatile or non-volatile silicone oils such as polydimethylsiloxanes (PDMS) and cyclodimethylsiloxanes or cyclomethicones, and fluoro oils or fluoro silicones, and also mixtures of these oils.

The composition may also comprise one or more mineral fillers and/or organic fillers in order to improve the sensory properties after rinsing and drying, especially as regards the softness and/or moisturization maintenance.

Fillers that may be mentioned include mineral fillers such as talc or magnesium silicate (particle size: 5 microns) sold under the name Luzenac 15 M00® by the company Luzenac, kaolin or aluminium silicate, for instance the product sold under the name Kaolin Supreme® by the company Imerys, or organic fillers such as starch, for instance the product sold under the name Amidon de Maïs B® by the company Roquette, Nylon microspheres such as those sold under the name Orgasol 2002 UD Nat Cos® by the company Atochem, microspheres based on expanded vinylidene chloride/acrylonitrile/methacrylonitrile copolymer containing isobutane, such as the products sold under the name Expancel 551 DE® by the company Expancel. Fibres, for instance nylon fibres (Polyamide 0.9 Dtex 0.3 mm sold by Etablissements Paul Bonte), or cellulose or "Rayon" fibres (Rayon Flock RCISE NOOO3 MO4® sold by the company Claremont Flock Corporation), may also be added to the composition of the invention.

Use will be made more particularly of a talc, such as the product sold under the name Luzenac 15 M00® by the company Luzenac.

The foaming compositions according to the invention may be used in cosmetics and dermatology, and they may especially constitute a cosmetic composition and in particular products for cleansing or for removing makeup from the skin (of the body, face or eyes), the scalp and/or the hair. They may be used for any type of skin (dry, normal, combination or greasy skin).

The compositions of the invention may especially be used as shower products; bath products; cleansing products for the hands; shampoos; makeup-removing products for the eyes and/or the face.

Another subject of the invention consists of the cosmetic use of the composition as defined above as a product for cleansing and/or for removing makeup from human keratin materials and more particularly the skin.

The compositions according to the invention may also be used for treating greasy skin, especially by adding thereto specific active agents for treating greasy skin, such as anti-seborrheic agents, for instance salicylic acid and derivatives thereof, azelaic acid, triclosan, tricarban, piroctone olamine or niacinamide (vitamin PP).

The compositions according to the invention have a final pH generally of between 3 and 10. Preferably, this pH is between 4 and 8. The pH may conventionally be adjusted to the desired value by adding a base (organic or mineral) to the composition, for example a mineral base such as sodium hydroxide, potassium hydroxide or aqueous ammonia, or an organic base such as a primary, secondary or tertiary (poly)amine, for instance monoethanolamine, diethanolamine, triethanolamine, isopropanolamine or 1,3-propanediamine, or alternatively by adding a mineral or organic acid, preferably a carboxylic acid, for instance citric acid.

The compositions of the invention may be packaged, according to the application chosen, in the form of a bottle, a tube, a pump-action bottle, an aerosol device or any other packaging method that is suitable for a liquid washing composition.

The cleansing compositions of the present invention packaged in aerosol devices may contain any propellant usually used for preparing aerosol compositions. Mention may be made in particular of hydrocarbon-based gases, for instance propane, n-butane or isobutane, and mixtures thereof; fluoro gases, for instance chlorodifluoromethane, dichlorodifluoromethane, difluoroethane, chlorodifluoroethane, dichlorotetrafluoroethane, etc., and mixtures thereof; fluorohydrocarbon gases; dimethyl ether and mixtures of dimethyl ether with one or more hydrocarbon-based gases; nitrogen, air and carbon dioxide and mixtures thereof may also be used as propellant gases in the present invention. Hydrocarbon-based gases containing from 2 to 6 carbon atoms are preferentially used in the present invention, and more particularly an isobutane/propane/n-butane mixture. The propellant gas(es) are present in the device in a proportion of from 0.1% to 15% by weight and more preferentially from 1% to 8% by weight relative to the total weight of the composition.

Finally, the present invention relates to the use of a crosslinked or non-crosslinked copolymer of methacrylic acid and of a C₁-C₄ alkyl acrylate in a liquid cleansing composition containing, in a cosmetically acceptable aqueous medium, at least one anionic surfactant and at least one electrolyte, for the purpose of improving the stability of the texture of said composition at low temperature.

The examples that follow serve to illustrate the invention without, however, being limiting in nature. The amounts indicated are weight percentages, unless otherwise mentioned, and the names of the compounds are the chemical names or the CTFA names (International Cosmetic Ingredient Dictionary and Handbook), depending on the case.

### EXAMPLES

### Example 1

| | |
|---|---|
| Glycerol | 3.0% |
| PEG-90M | 0.1% |
| Sodium Benzoate | 0.5% |
| Sodium Laureth Sulfate | 14.0% |
| Polyquaternium-7 | 1.25% |
| PEG-150 Pentaerythrityl tetrastearate & PEG-6 Caprylic/ Capric Glycerides (Crothix LQ®) | 0.6% |
| Salicylic Acid | 0.2% |
| Fragrance | 1.0% |
| Styrene/Acrylates copolymer(Opulyn 301 Opacifier®) | 0.5% |
| Acrylates Copolymer (Carbopol Aqua SF1®) | 5.0% |
| Sodium Hydroxide | 0.20% |
| Citric Acid | 0.1% |
| Cocoyl Betaine | 4.0% |
| Sodium Chloride | 0.5% |
| Water | qs 100 |

### Example 2

| | |
|---|---|
| Glycerol | 3.0% |
| PEG-90M | 0.1% |
| Sodium Benzoate | 0.5% |
| Sodium Laureth Sulfate | 14.0% |
| Polyquaternium-7 | 1.25% |
| PEG-150 Pentaerythrityl tetrastearate (Crothix PA®) | 0.3% |
| Salicylic Acid | 0.2% |
| Fragrance | 1.0% |
| Styrene/Acrylates copolymer(Opulyn 301 Opacifier®) | 0.5% |
| Acrylates Copolymer (Carbopol Aqua SF1®) | 5.0% |
| Sodium Hydroxide | 0.20% |
| Citric Acid | 0.1% |
| Cocoyl Betaine | 4.0% |
| Sodium Chloride | 0.5% |
| Water | qs 100 |

### 1) Self-evaluation test on children performed for 2 weeks on 50 individuals

A sensory test was performed for 2 weeks on 50 girls or boys from 3 to 12 years old, having sensitive skin on the body, with Examples 1 and 2.

The percentages of the people tested who approved the following sensory criteria are indicated in the following table.

| **QUALITY OF THE PRODUCT ON APPLICATION** | |
|---|---|
| The foam of this product is rich and unctuous | 96% |
| The texture melts on the skin on application | 88% |
| The foam of this product is soft | 96% |
| Has an unctuous, creamy texture | 96% |

| **EFFECTS ON THE SKIN** | |
|---|---|
| Leaves the skin soft | 98% |
| Leaves a powdery finish on the skin | 66% |
| Leaves the skin silky, velvety/ | 98% |

### 2) Self-evaluation test on adults performed for 4 weeks on 50 individuals

A sensory test was performed for 4 weeks on 13 men and 37 women from 24 to 49 years old, having sensitive skin on the face and the body, with Examples 1 and 2.

The percentages of the people tested who approved the following sensory criteria are indicated in the following table.

| **QUALITY OF THE PRODUCT ON APPLICATION** | |
|---|---|
| The foam of this product is rich and unctuous | 94% |
| The foam of this product is light | 92% |
| The texture melts on the skin on application | 88% |
| Is easy to apply and to spread | 100% |
| Mixes easily with water | 94% |
| Leaves a "powdery" finish on the skin | 62% |
| Has a rich texture | 98% |
| The foam envelops the skin with a pleasant sensation, has an enveloping texture | 92% |
| Has an unctuous, creamy texture | 96% |
| Is soft on application | 98% |

| **RINSING** | |
|---|---|
| Rinses off easily | 92% |
| Leaves a soft, protective film on the skin | 100% |

| **EFFECTS ON THE SKIN** | |
|---|---|
| Leaves the skin soft | *100%* |
| Leaves the skin as soft as velvet | *92%* |
| Affords a baby-skin effect | *92%* |
| Leaves the skin silky, velvety | *94%* |
| Leaves the skin softer | *94%* |
| Affords a baby-skin effect | *88%* |

### Examples 3 to 4

| **Ingredients** | **Example 3 (outside the invention)** | **Example 4 (outside the invention)** | **Example 5 (invention)** |
|---|---|---|---|
| Glycerol | 3 | 3 | 3 |
| PEG-90M | 0.1 | - | 0.1 |
| Sodium Benzoate | 0.5 | 0.5 | 0.5 |
| Sodium Laureth Sulfate | 14 | 14 | 14 |
| Polyquaternium-7 | 1.25 | 1.25 | 1.25 |
| PEG-150 Pentaerythrityl tetrastearate and PEG-6Caprylic/Capric Glycerides | - | 0.6 | 0.6 |
| Styrene/Acrylates copolymer | 0.5 | 0.5 | 0.5 |
| Acrylates Copolymer | - | 5 | 5 |
| Cocoyl Betaine | 4 | 4 | 4 |
| Water | qs 100 | qs 100 | qs 100 |

### 3) Hand test on 10 people:

Three shower gel formulations were tested on a panel of 10 people in order to evaluate, after application and drying of the skin, the "powdery finish" criterion according to the following protocol:
1/ The forearm is wetted, going beyond the crook of the elbow.
2/ 1 g of product is applied in a palm of the hand.
3/ A movement of three circles, with the other hand, is exerted on the hand which contains the product to generate foam.
4/ 15 to-and-fro actions are performed (from the inside of the wrist to the crook of the elbow),
   making the product foam between the hands on each passage.
5/ The washed parts (hands and forearm) are rinsed and dried.

| **Composition tested** | **Powdery finish** |
|---|---|
| **Example 3 (outside the invention)** | 5 |
| **Example 4 (outside the invention)** | 6 |
| **Example 5 (invention)** | 8 |

Composition 5 according to the invention has a better powdery finish than compositions 3 and 4.

## Claims

1. A composition comprising, especially in a physiologically acceptable medium:
(a) an aqueous phase; and
(b) at least one anionic foaming surfactant; and
(c) at least one amphoteric or zwitterionic foaming surfactant; and
(d) at least PEG-90M; and
(e) at least one of PEG-150 pentaerythrityl tetrastearate in isolated form or a mixture of PEG-150 pentaerythrityl tetrastearate, of PEG-6 caprylic/capric triglycerides and of water (50/20/30% by weight).; and
(f) a polymeric suspension agent, said polymeric suspension agent being chosen from:
- water-soluble polysaccharides;
- associative or non-associative crosslinked homopolymers and crosslinked copolymers of acrylic acid and/or methacrylic acid and/or esters thereof; and
- associative polyether-urethane polymers.

2. The composition as claimed in claim 1, in which the polyethylene glycol(s) are present in concentrations ranging from 0.01% to 1% and more preferentially from 0.05% to 0.5% by weight relative to the total weight of the composition.

3. The composition as claimed in any one of claims 1 to 2, in which the polyether or the polyether ester(s) of formula (II) are present in concentrations ranging from 0.01% to 1% by weight of active material and more preferentially from 0.05% to 0.5% by weight of active material relative to the total weight of the composition.

4. The composition as claimed in any one of claims 1 to 3, in which the anionic foaming surfactant is chosen from alkyl sulfates, alkyl ether sulfates, isethionates, amino acid derivatives, and mixtures thereof, and even more particularly chosen from alkyl ether sulfates and even more particularly sodium lauryl ether sulfate.

5. The composition as claimed in any one of claims 1 to 4, in which the anionic foaming surfactant(s) are present in concentrations ranging from 5% to 15% by weight of active material and more preferentially from 8% to 12% by weight relative to the total weight of the composition.

6. The composition as claimed in any one of claims 1 to 5, in which the foaming amphoteric or zwitterionic surfactant is chosen from betaines and alkyl amphoacetates, and mixtures thereof, and even more particularly chosen from cocoyl betaine and cocamidopropyl betaine, and mixtures thereof.

7. The composition as claimed in any one of claims 1 to 6, in which the foaming amphoteric or zwitterionic surfactant(s) are present in concentrations ranging from 1% to 10% by weight of active material and more preferentially from 1% to 5% by weight relative to the total weight of the composition.

8. The composition as claimed in claim 1, in which the polymeric suspension agent is chosen from:
- crosslinked copolymers comprising at least one methacrylic acid unit and at least one ethyl acrylate unit and more particularly in the form of an aqueous 30% dispersion;
- crosslinked non-associative copolymers of acrylic acid and/or methacrylic acid and/or an ester thereof comprising less than 6 carbon atoms, and more particularly in the form of an aqueous 28% dispersion.

9. The composition as claimed in any one of claims 1 to 8, in which the polymeric suspension agent is present in a concentration ranging from 0.1% to 10% by weight of active material relative to the total weight of the composition, more preferentially from 1% to 8% by weight and more particularly from 2% to 5% by weight relative to the total weight of the composition.

10. The composition as claimed in any one of claims 1 to 9, also comprising a polymeric quaternary ammonium salt.

11. The composition as claimed in any one of claims 1 to 10, also comprising one or more mineral fillers and/or organic fillers, more particularly a talc.

12. A cosmetic process for treating a keratin material, which consists in applying to the surface of said keratin material a composition as defined in any one of claims 1 to 11, followed by rinsing with water.

13. The cosmetic process for cleansing a keratin material, which consists in applying to the surface of the keratin material a composition as defined in any one of claims 1 to 11, followed by rinsing with water.

## Patentansprüche

1. Zusammensetzung, umfassend, insbesondere in einem physiologisch unbedenklichen Medium:
(a) eine wässrige Phase und
(b) mindestens ein anionisches schäumendes Tensid und
(c) mindestens ein amphoteres oder zwitterionisches schäumendes Tensid und
(d) mindestens PEG-90M und
(e) PEG-150-Pentaerythrityltetrastearat in isolierter Form und/oder eine Mischung von PEG-150-Pentaerythrityltetrastearat, PEG-6-Capryl/Caprintriglyceriden und Wasser (50/20/30 Gew.-%) und
(f) ein polymeres Suspendiermittel, wobei das polymere Suspendiermittel aus
- wasserlöslichen Polysacchariden,
- assoziativen oder nicht assoziativen vernetzten Homopolymeren und vernetzten Copolymeren von Acrylsäure und/oder Methacrylsäure und/oder Estern davon und
- assoziativen Polyetherurethanpolymeren ausgewählt ist.

2. Zusammensetzung nach Anspruch 1, in der das Polyethylenglykol bzw. die Polyethylenglykole in Konzentrationen im Bereich von 0,01 bis 1 Gew.-% und weiter bevorzugt von 0,05 bis 0,5 Gew.-%, bezogen auf das Gesamtgewicht der Zusammensetzung, vorliegen.

3. Zusammensetzung nach einem der Ansprüche 1 bis 2, in der der Polyether oder der Polyetherester bzw. die Polyetherester der Formel (II) in Konzentrationen im Bereich von 0,01 bis 1 Gew.-% Wirkstoff und weiter bevorzugt von 0,05 bis 0,5 Gew.-% Wirkstoff, bezogen auf das Gesamtgewicht der Zusammensetzung, vorliegen.

4. Zusammensetzung nach einem der Ansprüche 1 bis 3, in der das anionische schäumende Tensid aus Alkylsulfaten, Alkylethersulfaten, Isethionaten, Aminosäurederivaten und Mischungen davon und noch spezieller aus Alkylethersulfaten und noch spezieller aus Natriumlaurylethersulfat ausgewählt ist.

5. Zusammensetzung nach einem der Ansprüche 1 bis 4, in der das anionische schäumende Tensid bzw. die anionischen schäumenden Tenside in Konzentrationen im Bereich von 5 bis 15 Gew.-% Wirkstoff und weiter bevorzugt von 8 bis 12 Gew.-%, bezogen auf das Gesamtgewicht der Zusammensetzung, vorliegen.

6. Zusammensetzung nach einem der Ansprüche 1 bis 5, in der das schäumende amphotere oder zwitterichnische Tensid aus Betainen und Alkylamphoacetaten und Mischungen davon und noch spezieller aus Cocoylbetain und Cocamidopropylbetain und Mischungen davon ausgewählt ist.

7. Zusammensetzung nach einem der Ansprüche 1 bis 6, in der das schäumende amphotere oder zwitterionische Tensid bzw. die schäumenden amphoteren oder zwitterionischen Tenside in Konzentrationen im Bereich von 1 bis 10 Gew.-% Wirkstoff und weiter bevorzugt von 1 bis 5 Gew.-%, bezogen auf das Gesamtgewicht der Zusammensetzung, vorliegen.

8. Zusammensetzung nach Anspruch 1, in der das polymere Suspendiermittel aus
- vernetzten Copolymeren mit mindestens einer Methacrylsäure-Einheit und mindestens einer Ethylacrylat-Einheit und spezieller in Form einer wässrigen 30%igen Dispersion,
- vernetzten nicht assoziativen Copolymeren von Acrylsäure und/oder Methacrylsäure und/oder einem Ester davon mit mindestens 6 Kohlenstoffatomen und spezieller in Form einer wässrigen 28%igen Dispersion
ausgewählt ist.

9. Zusammensetzung nach einem der Ansprüche 1 bis 8, in der das polymere Suspendiermittel in einer Konzentration im Bereich von 0,1 bis 10 Gew.-% Wirkstoff, bezogen auf das Gesamtgewicht der Zusammensetzung, weiter bevorzugt von 1 bis 8 Gew.-% und spezieller von 2 bis 5 Gew.-%, bezogen auf das Gesamtgewicht der Zusammensetzung, vorliegt.

10. Zusammensetzung nach einem der Ansprüche 1 bis 9, die außerdem ein polymeres quaternäres Ammoniumsalz umfasst.

11. Zusammensetzung nach einem der Ansprüche 1 bis 10, die außerdem einen oder mehrere mineralische Füllstoffe und/oder organische Füllstoffe, spezieller einen Talk, umfasst.

12. Kosmetisches Verfahren zur Behandlung eines Keratinmaterials, bei dem man auf die Oberfläche des Keratinmaterials eine Zusammensetzung gemäß einem der Ansprüche 1 bis 11 aufbringt und anschließend mit Wasser spült.

13. Kosmetisches Verfahren zum Reinigen eines Keratinmaterials, bei dem man auf die Oberfläche des Keratinmaterials eine Zusammensetzung gemäß einem der Ansprüche 1 bis 11 aufbringt und anschließend mit Wasser spült.

## Revendications

1. Composition comprenant, en particulier dans un milieu physiologiquement acceptable :
(a) une phase aqueuse ; et
(b) au moins un tensioactif moussant anionique ; et
(c) au moins un tensioactif moussant amphotère ou zwitterionique ; et
(d) au moins PEG-90M ; et
(e) au moins l'un parmi PEG-150-tétrastéarate de pentaérythrityle sous forme isolée ou un mélange de PEG-150-tétrastéarate de pentaérythrityle, de PEG-6-triglycérides capryliques/capriques et d'eau (50/20/30 % en poids) ; et
(f) un agent de suspension polymère, ledit agent de suspension polymère étant choisi parmi :
- des polysaccharides hydrosolubles ;
- des homopolymères et copolymères réticulés associatifs ou non associatifs d'acide acrylique et/ou d'acide méthacrylique et/ou des esters de ceux-ci ; et
- des polymères de polyéther-uréthane associatifs.

2. Composition selon la revendication 1, dans laquelle le (s) polyéthylène glycol(s) sont présents à des concentrations dans la plage de 0,01 % à 1 % et plus préférablement de 0,05 % à 0,5 % en poids par rapport au poids total de la composition.

3. Composition selon l'une quelconque des revendications 1 à 2, dans laquelle le polyéther ou le(s) ester(s) de polyéther de formule (II) sont présents à des concentrations dans la plage de 0,01 % à 1 % en poids de matériau actif et plus préférablement de 0,05 % à 0,5 % en poids de matériau actif par rapport au poids total de la composition.

4. Composition selon l'une quelconque des revendications 1 à 3, dans laquelle le tensioactif moussant anionique est choisi parmi des sulfates d'alkyle, des sulfates d'éther d'alkyle, des iséthionates, des dérivés d'acide aminé, et des mélanges de ceux-ci, et encore plus particulièrement choisis parmi des sulfates d'éther d'alkyle et encore plus particulièrement le sulfate d'éther laurylique de sodium.

5. Composition selon l'une quelconque des revendications 1 à 4, dans laquelle le(s) tensioactif(s) moussant(s) anionique(s) sont présents à des concentrations dans la plage de 5 % à 15 % en poids de matériau actif et plus préférablement de 8 % à 12 % en poids par rapport au poids total de la composition.

6. Composition selon l'une quelconque des revendications 1 à 5, dans laquelle le tensioactif moussant amphotère ou zwitterionique est choisi parmi des bétaïnes et des amphoacétates d'alkyle, et des mélanges de ceux-ci, et encore plus particulièrement choisi parmi la cocoyl-bétaïne et la cocamidopropyl-bétaïne, et des mélanges de celles-ci.

7. Composition selon l'une quelconque des revendications 1 à 6, dans laquelle le(s) tensioactif(s) moussant(s) amphotère(s) ou zwitterionique(s) sont présents à des concentrations dans la plage de 1 % à 10 % en poids de matériau actif et plus préférablement de 1 % à 5 % en poids par rapport au poids total de la composition.

8. Composition selon la revendication 1, dans laquelle l'agent de suspension polymère est choisi parmi :
- des copolymères réticulés comprenant au moins un motif d'acide méthacrylique et au moins un motif d'acrylate d'éthyle et plus particulièrement sous la forme d'une dispersion aqueuse à 30 % ;
- des copolymères non associatifs réticulés d'acide acrylique et/ou d'acide méthacrylique et/ou un ester de ceux-ci comprenant moins de 6 atomes de carbone, et plus particulièrement sous la forme d'une dispersion aqueuse à 28 %.

9. Composition selon l'une quelconque des revendications 1 à 8, dans laquelle l'agent de suspension polymère est présent à une concentration dans la plage de 0,1 % à 10 % en poids de matériau actif par rapport au poids total de la composition, plus préférablement de 1 % à 8 % en poids et plus particulièrement de 2 % à 5 % en poids par rapport au poids total de la composition.

10. Composition selon l'une quelconque des revendications 1 à 9, comprenant en outre un sel d'ammonium quaternaire polymère.

11. Composition selon l'une quelconque des revendications 1 à 10, comprenant en outre une ou plusieurs charges minérales et/ou charges organiques, plus particulièrement un talc.

12. Procédé cosmétique de traitement d'un matériau kératinique, qui consiste à appliquer sur la surface dudit matériau kératinique une composition telle que définie dans l'une quelconque des revendications 1 à 11, suivie d'un rinçage avec de l'eau.

13. Procédé cosmétique de nettoyage d'un matériau kératinique, qui consiste à appliquer sur la surface du matériau kératinique une composition telle que définie dans l'une quelconque des revendications 1 à 11, suivie d'un rinçage avec de l'eau.
